# EUROPEAN PATENT SPECIFICATION

(11) **EP 2 649 909 B1**
(45) Date of publication and mention of the grant of the patent: **15.08.2018**
(21) Application number: 13163725.8
(22) Date of filing: 15.04.2013
(51) Int. Cl.: A47C 27/14, A47G 9/10, A61F 5/56

(54) **An improved pillow**
Verbessertes Kissen
Oreiller amélioré

(30) Priority: 14.04.2012 GB 201206621
(43) Date of publication of application: 16.10.2013
(73) Proprietor: Mammoth Sport Limited, Cramlington, Northumberland NE23 1WG (GB)
(72) Inventor: Tuton, John, Cramlington, Northumberland NE23 1WG (GB)
(74) Representative: Archer, Graham John

(56) References cited:
- EP-A1- 0 286 248
- WO-A1-95/26154
- DE-A1- 4 001 991
- DE-A1- 10 046 412
- DE-A1-102009 060 628
- GB-A- 1 321 716
- JP-A- 2003 339 504
- US-A- 5 459 896

## Description

The present invention relates to an improved pillow.

JP2003339504A, WO95/26154 and GB131716A show examples of pillows.

Pillows are used to support the head of a person who is lying down. A conventional pillow generally consists of a single compartment in which a filling material is disposed.
Conventional pillows function adequately to some extent, however they do not always provide adequate head and/or neck support for the user, which can lead to discomfort and potential posture problems.
Moreover, on account of the types of material often used as the filling for the compartment, the pillow does not always provide an adequately cool support surface to the user, which can lead to discomfort.
An object of the present invention is to provide an improved pillow, which overcomes or at least alleviates, at least the above-mentioned disadvantages associated with the prior art.
According to the present invention there is provided a pillow as defined in claim 1.
This provides the advantage that the pillow is more pliable. To elaborate, in being arranged so that they have a smaller surface area where they are attached to the body of the pillow than at their upper surface which is presented to the user, the raised portions are able to depress more easily when the user lies down on the pillow. To elaborate, the raised portions are made more compressible by virtue of their physical shape; that is, the fact that they are smaller at their base than at their upper surface; as well as by virtue of the material they are composed of. The pillow is made from resiliently deformable material.
Preferably, the pillow has a substantially cuboid form.
Preferably, the body has an upper surface and a lower surface, each surface comprising a plurality of said raised portions.
Preferably, the upper surface of the body is substantially rectangular.
Preferably, the upper surface of the body is planar.
Alternatively, the upper surface of the body is curvilinear.
Preferably, the lower surface of the body is rectangular.
Preferably, the lower surface of the body is planar.
Preferably, said upper surface and said lower surface are castellated.
This provides the advantage that the pillow is more pliable. In this way, a denser material can be used as the material for the pillow, whilst still affording the pillow with pliability, which is desirable for the user. In using a denser material for the pillow, this provides the advantage that the pillow is more hard-wearing.
Further, by castellating the body of the pillow, the pillow reacts more effectively to pressure changes in order to contour to the mass of the head and neck of the user. This in turn, reduces pressure points and creates a wider surface area for better pressure distribution and support for the head, neck and shoulders of the user, without the pillow collapsing.
This provides the further advantage that the circulation of air throughout the pillow is facilitated, the raised portions facilitating the flow of air throughout the pillow. As a result, the pillow feels cooler to the user. The improved circulation of air throughout the pillow also facilitates the dispersion of alergens and other particles such as dust, bed bugs, faeces and pollen. Said raised portions are made from resiliently deformable material. The pillow comprises a groove disposed around at least a portion of the periphery of the body.
This provides the advantage that the pillow is more pliable. Said groove is continuous and disposed between said upper surface and said lower surface.
Preferably, said resiliently deformable material comprises flexible polyurethane foam.
Preferably, said flexible polyurethane foam is combustion modified.
Preferably, said flexible polyurethane foam is a combustion modified polyether.
Preferably, said flexible polyurethane foam is a combustion modified visco-elastic material.
Preferably, said flexible polyurethane foam is a combustion modified highly resilient material.

Preferably, said flexible polyurethane foam is combustion modified synthetic latex.
Preferably, said pillow further comprises a cover.
Preferably, said cover is made from fabric.
Preferably, said cover comprises poly fibre.
This provides the advantage that the pillow feels more comfortable to the user, in particular in the early stages of use; that is, when the user first lies down on the pillow.
Preferably, said raised portions are substantially rectangular in cross section.
Preferably, said raised portions are substantially square in cross section.
Alternatively, said raised portions are substantially trapezoidal in cross section.
Said raised portions may be substantially reverse trapezoidal in cross section. Said raised portions are narrower at their base than at their upper surface.
It is to be appreciated that in being narrower at their base, the raised portions are narrower at that part which is attached to the body of the pillow, than at the surface which is presented to the user when they lie on the pillow.

In accordance with a second aspect not according to the present invention, there is provided a pillow comprising a body, characterised in that the body comprises a groove disposed around at least a portion of its periphery.

Preferably, said groove is continuous.
Preferably, said pillow comprises an upper surface which in use provides a support surface for a user, and a lower surface, wherein said groove is disposed between said upper surface and said lower surface.

In accordance with a third aspect not according to the present invention, there is provided a pillow comprising a body, said body comprising a central portion and first and second portions disposed at either side of said central portion, characterised in that at least a portion of the central portion comprises a material which is less deformable than the material of the first and second portions.
Preferably, said central portion is elongate and extends substantially across the width of the pillow.
Said central portion may extend across the height of the pillow.
Preferably, said central portion is disposed on the upper surface of the body.
Alternatively, said central portion may be disposed inside the body of the pillow.
This provides the advantage that a user is discouraged from resting their head towards the centre of the pillow, with the central portion effectively functioning as a "roll bar" to encourage the user of the pillow to lie on their side. It is known that when a person lies on their side whilst sleeping, they are less likely to snore. Accordingly, in encouraging a user to lie on their side, the pillow helps to alleviate snoring.
To elaborate, when a user rests their head on the central portion of the pillow, they feel uncomfortable in view of the fact that the central portion is less deformable; that is, harder, and so they turn on their side and instead rest their head on one or the other of the two portions on either side of the central portion, to make themselves more comfortable.

Preferably, said central portion comprises foam.
Alternatively, said central portion comprises polyfibre.
Preferably, said first and second portions either side of the central portion comprise foam.
Alternatively, said first and second portions either side of the central portion comprise polyfibre.
Preferred embodiments of the present invention will now be described, by way of example only and not in any limitative sense, with reference to the accompanying drawings in which: -
Figure 1 shows a perspective view from the front and above, of a pillow;
Figure 2 shows a view from above of the embodiment of Figure 1;
Figure 3 shows a perspective view from the front and above, of a second example, showing hidden detail;
Figure 4 shows a perspective view from the side, the front and above, of a thirdexample;
Figure 5 shows a side view of a pillow in accordance with a fourth example;
Figure 6 shows a side view of a first embodiment of a raised portion which is narrower at its base than at its upper surface;
Figure 7 shows a side view of a second embodiment of a raised portion which is narrower at its base than at its upper surface;
Figure 8 shows a side view of a third embodiment of a raised portion which is narrower at its base than at its upper surface;
Figure 9 shows a perspective view from the front and above of a pillow in accordance with a fifth example and an embodiment of the present invention;
Figure 10 shows a perspective view from the front, above and one side, of a pillow in accordance with a sixth example showing hidden detail;
Figure 11 shows a front sectional view of the pillow of Figure 10;
Figure 12 shows a side sectional view of the pillow of Figure 10;
Figure 13 shows a perspective view from the end and top of a pillow in accordance with a seventh example and
Figure 14 shows a perspective view from the front, above and one side, of a pillow in accordance with an eighth example showing hidden detail.
With reference to Figures 1 and 2, a first example of a pillow is represented generally by reference numeral 1.
The pillow comprises a body 3, comprising a plurality of raised portions 5 disposed evenly over the surface of the body 3, in a castellated formation.
The pillow 1 is made from resiliently deformable material and as can be clearly seen from Figure 1 in particular, has a substantially cuboid form.

The body 3 has an upper surface 3a and a lower surface 3b, whereby in use, the user rests their head on the upper surface 3a. The upper 3a and lower 3b surfaces are substantially rectangular. The raised portions 5 are substantially evenly dispersed over the surfaces 3a and 3b.

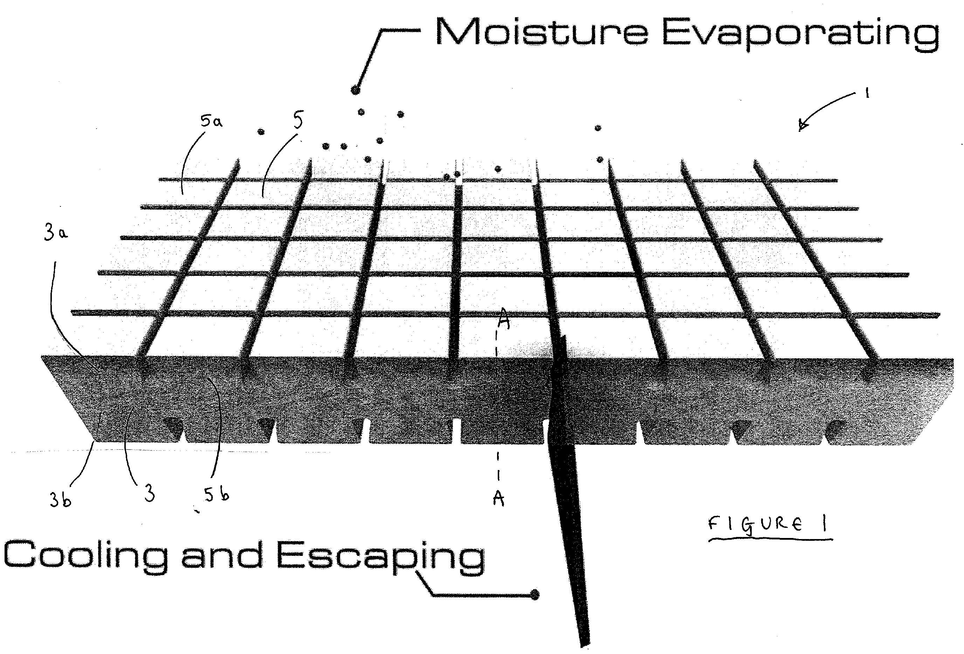

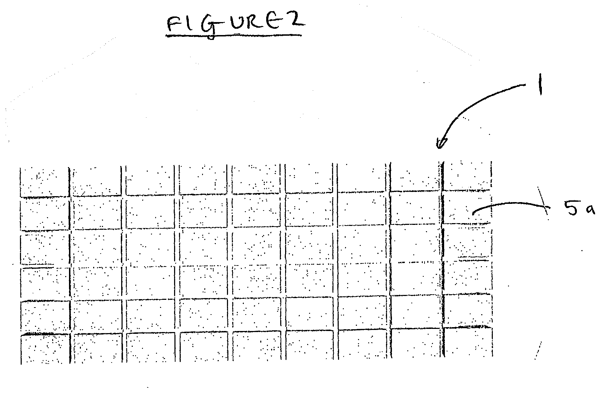

Again as can be clearly seen from Figures 1 and 2, as a result of the raised portions 5, the body 3 is castellated, which provides numerous advantages including making the pillow 1 more pliable. In this way, a denser material can be used whilst still affording the pillow 1 with pliability, which is desirable for the user. In using a denser material for the pillow 1, this provides the advantage that the pillow 1 is more hard-wearing.

The pillow 1 may be made from any suitable resiliently deformable material, for example, combustion modified flexible polyurethane foam such as combustion modified polyether, combustion modified visco-elastic material, combustion modified highly resilient material or combustion modified synthetic latex.

As can be clearly seen from Figure 2 in particular, the raised portions 5 each have a substantially rectangular upper surface 5a, which is presented to the user.

As can be clearly seen from the Figures, the raised portions 5 have a trapezoidal profile in cross section along line A-A. In this way, the raised portions 5 are narrower at their base 5b than at their upper surface 5a. To elaborate, the surface area of the base 5b of each raised portion 5 is less than the surface area of the upper surface 5a of each raised portion 5.

This provides the advantage that the pillow 1 is more pliable. To elaborate, in being arranged so that they are smaller at their base 5b, the raised portions 5 are able to depress more easily when the user lies down on the pillow 1, by means of their structure as opposed to the specific material they are made from.

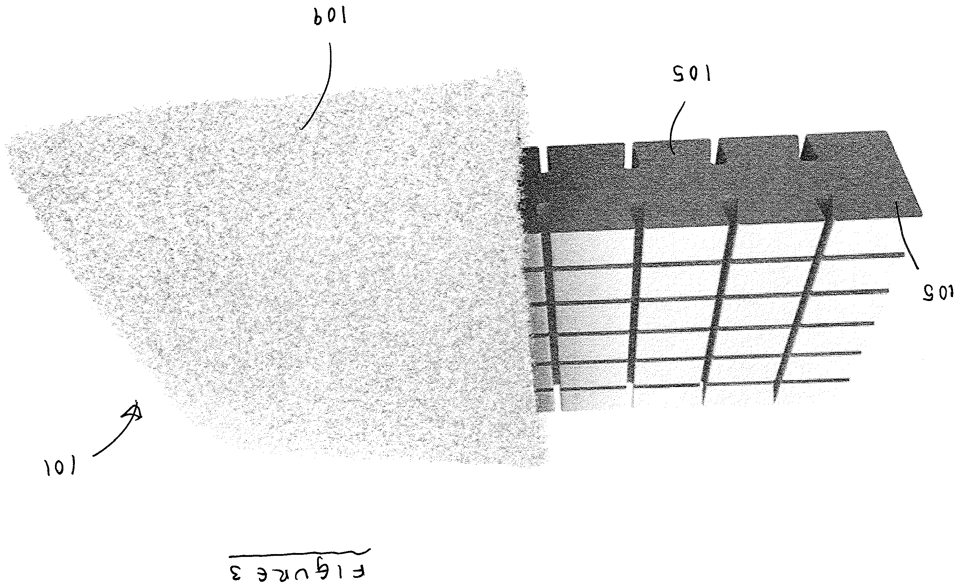

Turning now to Figure 3, a second example of a pillow is represented generally by reference numeral 101.
The interior of the pillow 101 is identical to the form of the pillow 1 represented by Figures 1 and 2. However, the pillow 101 differs from that of Figures 1 and 2 in that it further includes a cover 109, which in this example is made from poly fibre. In this example, the poly fibre cover 109 completely surrounds the interior of the pillow 101, effectively filling in the gaps between the raised portions 105 of the pillow 101 and providing additional padding around the interior of the pillow 101.
In having a poly fibre cover 109, this provides the advantage that the pillow 101 feels more comfortable to the user, in particular in the early stages of use; that is, when the user first lies down on the pillow 101.
It is to be appreciated that the poly fibre cover 109 could be applied to any of the embodiments of the present invention.

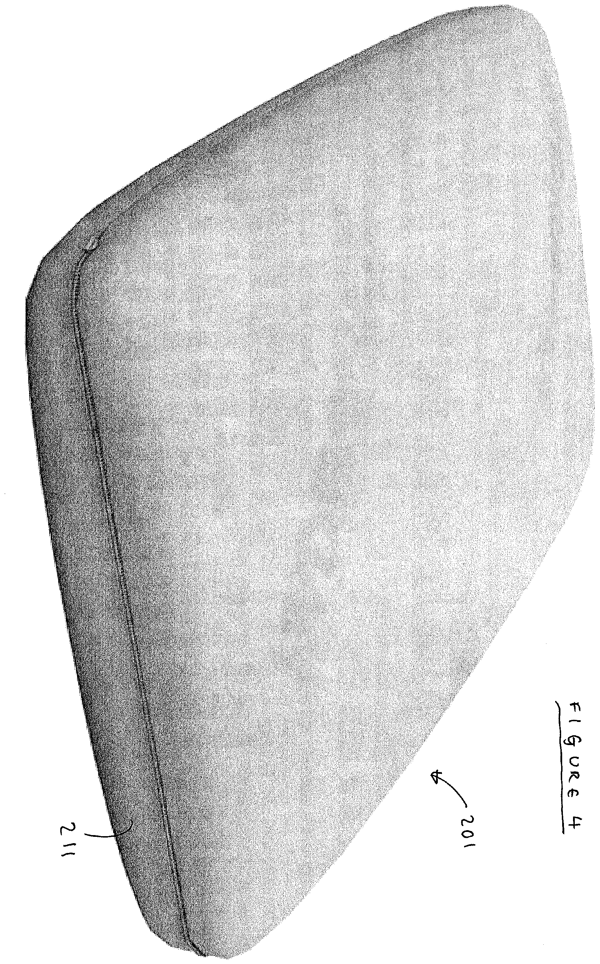

Turning now to Figure 4, a third example of a pillow is represented generally by reference numeral 201.
The interior of the pillow 201 is identical to the form of the pillow 1 represented by Figure 3. However, the pillow 201 differs from that of Figure 3 in that it further includes an outer cover 211, which in this example is made from fabric such as cotton or polycotton, surrounding its interior. The outer cover 211 covers the poly fibre cover of the pillow 201, and provides a protective cover around the interior of the pillow 201.
It is to be appreciated that the outer cover 211 could be applied to any of the embodiments of the present invention.

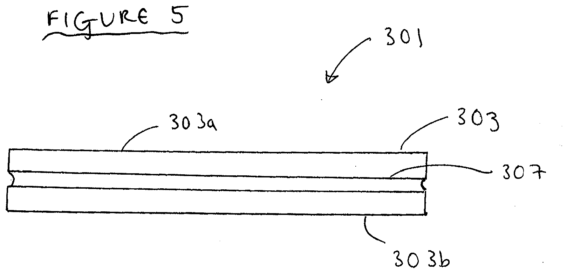

Turning now to Figure 5, a fourth example of a pillow is represented generally by reference numeral 301.

The pillow 301 is made from resiliently deformable material and as can be clearly seen from Figure 5, has a substantially cuboid form.
The pillow 301 comprises a body 303 having an upper surface 303a and a lower surface 303b along with a continuous groove 307 around the periphery of the body 303. The groove 307 is disposed mid-way between the upper surface 303a and the lower surface 303b. This provides the advantage that the pillow 301 is more pliable to the user and reacts more effectively to pressure changes in order to contour to the mass of the head and neck of the user. This in turn, reduces pressure points and creates a wider surface area for better pressure distribution and support for the head, neck and shoulders of the user, without the pillow 301 collapsing.
This provides the further advantage that the circulation of air throughout the pillow 301 is facilitated, with the result that the pillow 301 feels cooler to the user. The improved circulation of air throughout the pillow 301 also facilitates the dispersion of alergens and other particles such as dust, bed bugs, faeces and pollen.
The pillow 301 may be made from any suitable resiliently deformable material, for example, combustion modified flexible polyurethane foam such as combustion modified polyether, combustion modified visco-elastic material, combustion modified highly resilient material or combustion modified synthetic latex.
It is to be appreciated that the pillow 301 could additionally include a poly fibre cover similar to the cover 109 of Figure 3, and / or an outer cover similar to the cover 211 of Figure 4.

It is also to be appreciated that, in an embodiment of the invention, the pillow 301 could additionally include the raised portions represented in relation to the examples of Figures 1 to 4.

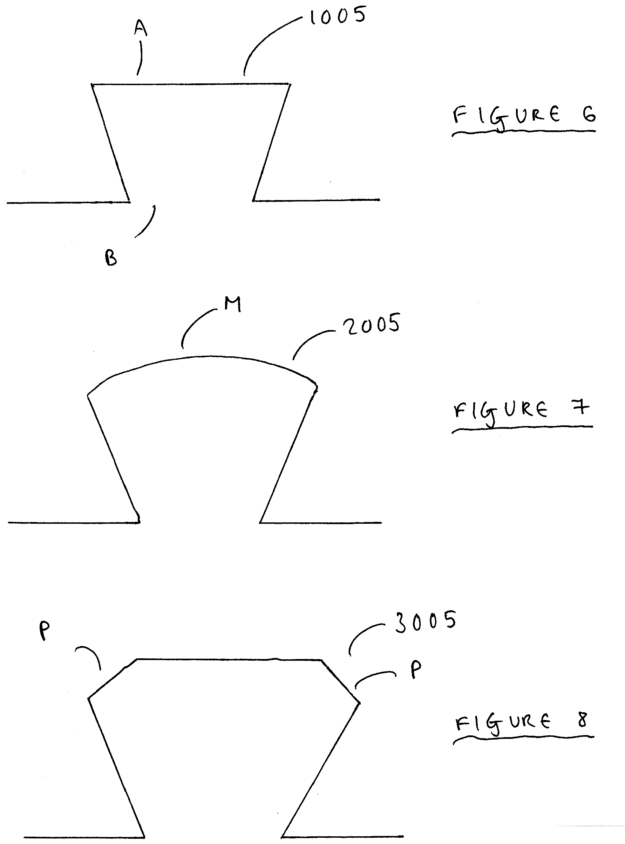

It is also to be appreciated that the continuous groove 307 could be applied to any of the examples. Turning now to Figures 6 to 8, various embodiments of raised portions which may be applied to any of the embodiments of the pillow are shown. For example, in Figure 6, the raised portion 1005 is reverse trapezoidal in cross section, being smaller at the base than at the upper surface, with two substantially parallel surfaces A and B.
In Figure 7, the raised portion 2005, whilst being smaller at the base than at the upper surface in a similar fashion to the embodiment of Figure 6, has a curved upper surface M.
In Figure 8, the raised portion 3005, whilst being smaller at the base than at the upper surface in a similar fashion to the embodiment of Figure 6, has a substantially flat upper surface but with chamfered corners P.
It is to be appreciated that the differently shaped raised portions of Figures 6 to 8 could be applied to any of the embodiments of the present invention.

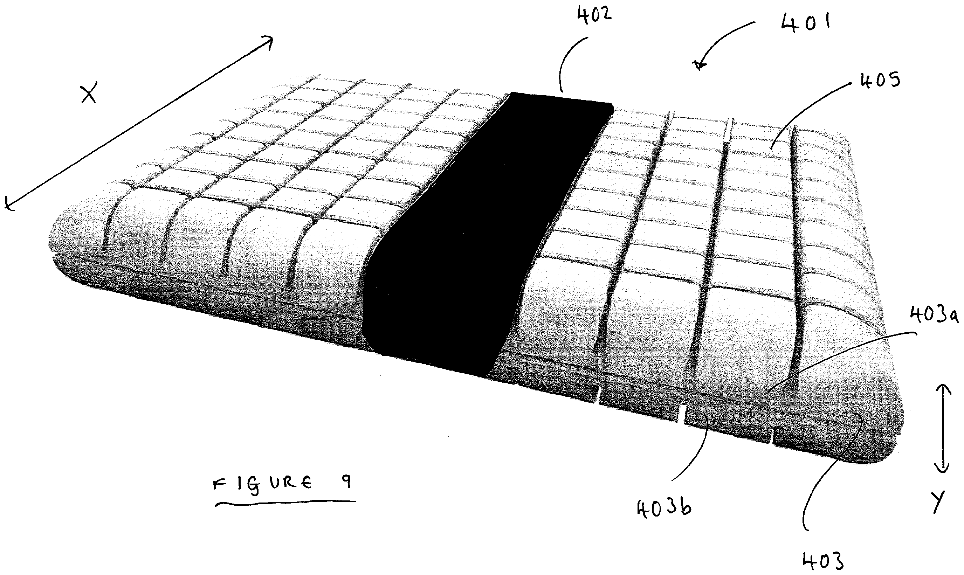

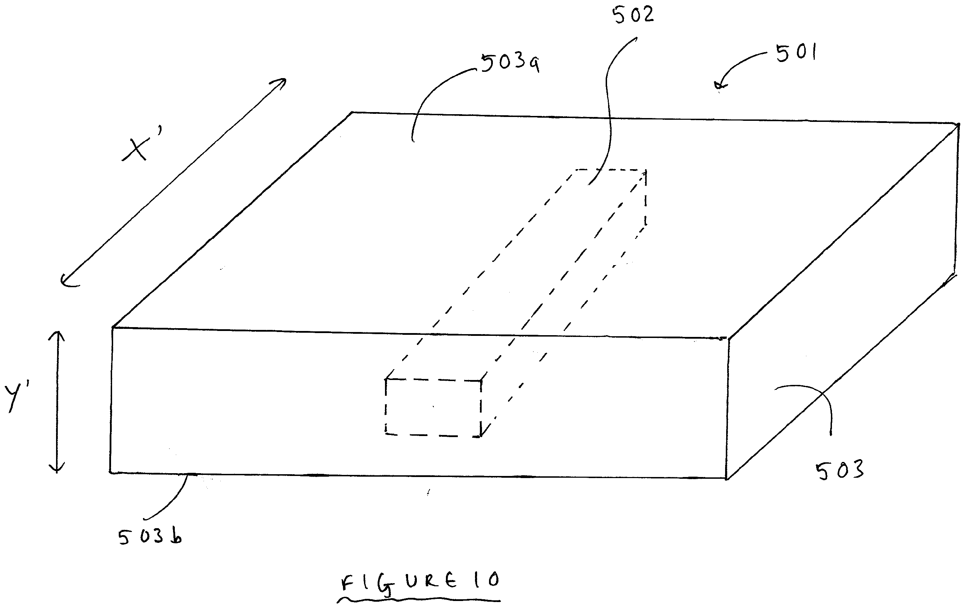

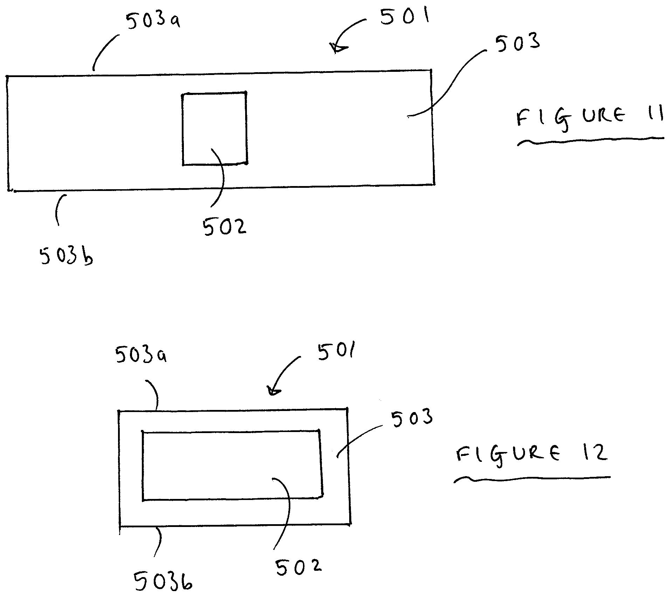

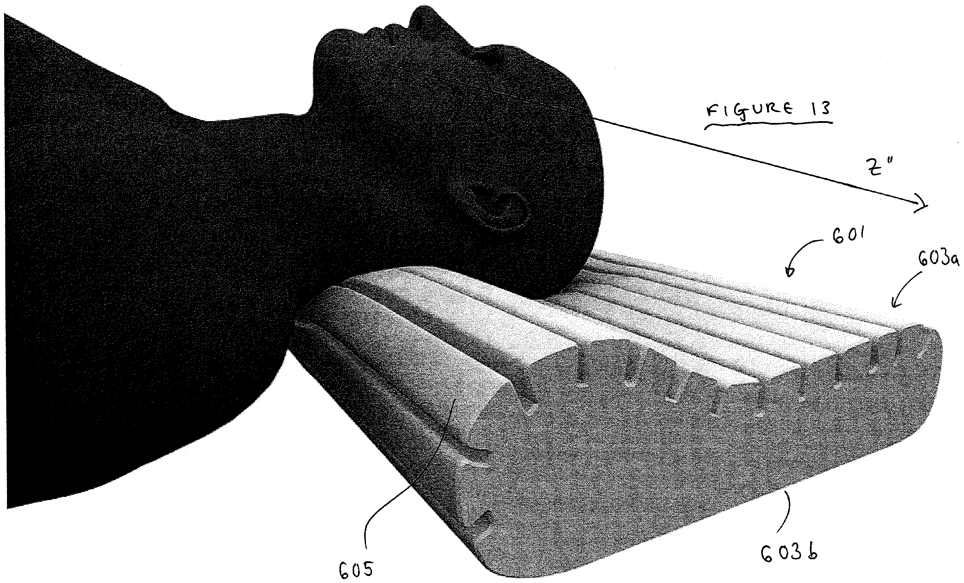

Turning now to Figure 9, a fifth example of a pillow and an embodiment of the invention is represented generally by reference numeral 401.
The pillow 401 is made from resiliently deformable material and has a substantially cuboid form.
The pillow 401 comprises a body 403 having an upper surface 403a and a lower surface 403b, with a plurality of raised portions 405 disposed across the upper 403a and lower 403b surface of the pillow 401.
The pillow 401 may be made from any suitable resiliently deformable material, for example, combustion modified flexible polyurethane foam such as combustion modified polyether, combustion modified visco-elastic material, combustion modified highly resilient material or combustion modified synthetic latex.
The pillow 401 comprises a central portion in the form of an elongate portion 402 extending across the entire width X of the central portion of the pillow 401 and across the entire depth Y of the pillow 401.
The elongate portion 402 is made from a less deformable material than the remainder of the pillow 402, and in this way, the central portion of the pillow 401 presents a harder and therefore more uncomfortable surface to the user of the pillow 401. As a result, in the event that a user rests their head in the centre of the pillow 401, they tend to roll away from the centre of the pillow 401, onto their side, and towards either one of the portions to each side of the elongate portion 402. In view of the fact that the pillow 401 causes the user to roll onto their side, snoring is alleviated.
It is to be appreciated that alternatively, the raised portions 405 could be absent from the pillow 401, though not an embodiment of the invention. It is also to be appreciated that the central elongate portion 402 could be applied to any of the examples. Turning now to Figures 10 to 12, a sixth embodiment of a pillow is represented generally by reference numeral 501.
The pillow 501 is made from resiliently deformable material and has a substantially cuboid form.
The pillow 501 comprises a body 503 having an upper surface 503a and a lower surface 503b and is made from any suitable resiliently deformable material, for example, combustion modified flexible polyurethane foam such as combustion modified polyether, combustion modified visco-elastic material, combustion modified highly resilient material or combustion modified synthetic latex.
The pillow 501 further comprises a further portion in the form of an elongate portion 502 extending across most, but not all, of the entire width X' of the pillow 501. As can be clearly seen from Figure 10, the elongate portion 502 does not extend across the entire depth Y' of the pillow 501 and instead is disposed within the pillow 501 such that it is completely surrounded by the remainder of the pillow 501, in effect, "floating" inside the pillow. In this way, the central portion of the pillow 501 still presents a harder and therefore more uncomfortable central portion to the user of the pillow 501, but not so much as with the embodiment of Figure 9, since the hard elongate portion 502 is surrounded by the softer remaining portion of the pillow 501.
As with the embodiment of Figure 9, as a result of the harder central portion, in the event that a user rests their head in the centre of the pillow 501, they tend to roll away from the centre of the pillow 501, onto their side, and towards either one of the portions to each side of the elongate portion 502. In view of the fact that the pillow 501 causes the user to roll onto their side, snoring is alleviated.
It is also to be appreciated that the pillow 501 could further comprise raised portions of the type disclosed with reference to the other examples. It is also to be appreciated that the central elongate portion 502 could be applied to any of the examples. Turning now to Figure 13, a seventh embodiment of a pillow is represented generally by reference numeral 601.
The pillow 601 is made from resiliently deformable material and has a generally organic form.

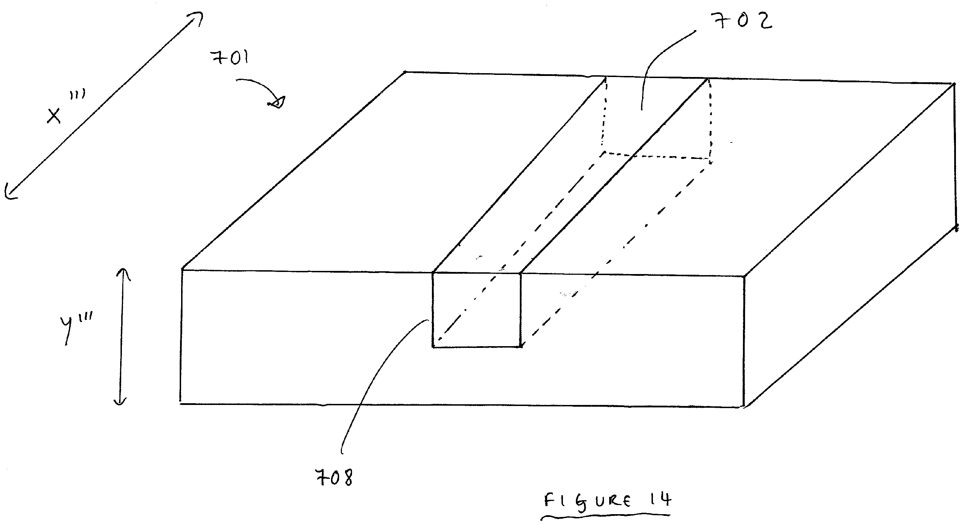

In particular, the pillow 601 comprises a body 603 having a curvilinear upper surface 603a and a substantially flat lower surface 603b. The pillow is made from any suitable resiliently deformable material, for example, combustion modified flexible polyurethane foam such as combustion modified polyether, combustion modified visco-elastic material, combustion modified highly resilient material or combustion modified synthetic latex.
The pillow 601 comprises a plurality of raised portions 605 disposed over the curvilinear upper surface 603a of the pillow 601. However, the raised portions 605 of the pillow 601 are different from those of the other examples, in that they are elongate, extending across the length Z" of the pillow 601 such that a raised portions 605 effectively spans the length Z" of the pillow 601.
It is also to be appreciated that the curvilinear upper surface 603a could be applied to any of the examples. Turning now to Figure 14, an eighth example of a pillow is represented generally be reference numeral 701.

The pillow 701 is identical to the example of Figures 10 to 12, save for the configuration of the elongate portion 702. In this example the elongate portion 702 extends across the entire width X"" of the central portion of the pillow 701. However, the elongate portion 702 does not extend across the entire depth Y"" of the pillow 701 and instead is disposed within a channel 708 formed in the upper portion of the pillow 701 such that the upper surface of the elongate portion 702 is presented to the user when they rest their head on the pillow 701.

As with the examples of Figures 9 to 12, in this way, the central portion of the pillow 701 presents a harder and therefore more uncomfortable surface to the user of the pillow 701, with the result that the user tends to roll away from the centre of the pillow 701, onto their side, and towards either one of the portions to each side of the elongate portion 702.

In view of the fact that the pillow 701 causes the user to roll onto their side, snoring is alleviated.
It is also to be appreciated that the pillow 701 could alternatively further comprise raised portions of the type described with reference to the other examples. It is also to be appreciated that the upper surface of the elongate portion 702 could be elevated from the remainder of the pillow 701, standing proud from the remainder of the pillow, such that the depth of the pillow 701 at the central portion is greater than the depth of the pillow at the first and second portions.
It is also to be appreciated that the central elongate portion 702 could be applied to any of the examples. It will be appreciated by persons skilled in the art that the above embodiments have been described by way of example only, and not in any limitative sense, and that various alterations and modifications are possible without departing from the scope of the invention as defined by the appended claims.

## Claims

1. A pillow having an upper surface which in use provides a support surface for a user, and a lower surface, the pillow being made of resiliently deformable material with a body which comprises a plurality of raised portions disposed over at least a portion of its surface, each said raised portion comprising an upper surface which in use, provides a surface upon which a user rests their head, wherein the surface area of that portion of at least one said raised portion which is attached to the body of the pillow is less than the surface area of the upper surface of said at least one raised portion, the body comprising, in addition to the raised portions, a continuous groove disposed around its periphery, the groove being disposed between upper and lower surfaces of said body.

2. A pillow as claimed in claim 1, wherein said upper and lower surfaces of said body each comprise a plurality of said raised portions.

3. A pillow as claimed in any one of the previous claims, wherein said raised portions are substantially reverse trapezoidal in cross section

4. A pillow as claimed in any one of the previous claims, wherein said raised portions are narrower at that portion of at least one said raised portion which is attached to the body of the pillow, than at their upper surface.

5. A pillow as claimed in any one of the previous claims, wherein said pillow further comprises a cover made from fabric.

6. A pillow as claimed in any one of the previous claims, wherein said body comprises a central portion and first and second portions disposed at either side of said central portion, **characterised in that** at least a portion of the central portion comprises a material which is less deformable than the material of the first and second portions.

7. A pillow as claimed in claim 6, wherein said central portion is elongate and extends substantially across the width of the pillow.

8. A pillow as claimed in any one of claims 6 or 7, wherein said central portion extends across the height of the pillow.

9. A pillow as claimed in any one of claims 6 to 8, wherein said central portion is disposed on the upper surface of the body.

10. A pillow as claimed in any one of claims 6 to 9, wherein said central portion is disposed inside the body of the pillow.

## Patentansprüche

1. Kissen mit einer oberen Oberfläche, die im Gebrauch eine Auflagefläche für einen Benutzer bereitstellt, und einer unteren Oberfläche, wobei das Kissen aus elastisch verformbarem Material mit einem Körper hergestellt ist, der mehrere erhabene Teile aufweist, die auf wenigstens einem Teil seiner Oberfläche angeordnet sind, wobei jeder erhabene Teil eine obere Oberfläche aufweist, die im Gebrauch eine Oberfläche bereitstellt, auf der der Benutzer seinen Kopf ruhen lässt, wobei der Oberflächeninhalt desjenigen Teils von wenigstens einem genannten erhabenen Teil, der an den Körper des Kissens angefügt ist, kleiner ist als der Oberflächeninhalt der oberen Oberfläche des genannten wenigstens einen erhabenen Teils, wobei der Körper zusätzlich zu den erhabenen Teilen eine kontinuierliche Nut aufweist, die um seinen Umfang angeordnet ist, wobei die Nut zwischen der oberen und der unteren Oberfläche des genannten Körpers angeordnet ist.

2. Kissen nach Anspruch 1, wobei die genannte obere und untere Oberfläche des genannten Körpers jeweils mehrere der genannten erhabenen Teile aufweisen.

3. Kissen nach einem der vorhergehenden Ansprüche, wobei die genannten erhabenen Teile im Querschnitt im Wesentlichen umgekehrt trapezförmig sind.

4. Kissen nach einem der vorhergehenden Ansprüche, wobei die genannten erhabenen Teile an demjenigen Teil von dem wenigstens einen genannten erhabenen Teil, der an den Körper des Kissens angefügt ist, schmäler sind als an ihrer oberen Oberfläche.

5. Kissen nach einem der vorhergehenden Ansprüche, wobei das genannte Kissen ferner einen aus einem Flächengebilde hergestellten Bezug aufweist.

6. Kissen nach einem der vorhergehenden Ansprüche, wobei der genannte Körper einen mittigen Teil und einen ersten und einen zweiten Teil aufweist, die auf beiden Seiten des genannten mittigen Teils angeordnet sind, **dadurch gekennzeichnet, dass** wenigstens ein Teil des mittigen Teils ein Material aufweist, das weniger verformbar ist als das Material des ersten und des zweiten Teils.

7. Kissen nach Anspruch 6, wobei der genannte mittige Teil länglich ist und sich im Wesentlichen über die Breite des Kissens erstreckt.

8. Kissen nach einem der Ansprüche 6 oder 7, wobei der genannte mittige Teil sich über die Höhe des Kissens erstreckt.

9. Kissen nach einem der Ansprüche 6 bis 8, wobei der genannte mittige Teil auf der oberen Oberfläche des Körpers angeordnet ist.

10. Kissen nach einem der Ansprüche 6 bis 9, wobei der genannte mittige Teil im Inneren des Körpers des Kissens angeordnet ist.

## Revendications

1. Oreiller comprenant une surface supérieure qui, lorsqu'utilisée, constitue une surface d'appui pour un utilisateur, et une surface inférieure, l'oreiller étant fabriqué dans une matière élastiquement déformable et dont le corps comporte une pluralité de parties surélevées disposées sur au moins une portion de sa surface, chaque dite partie surélevée comprenant une surface supérieure qui, lorsque utilisée, constitue une surface sur laquelle repose la tête d'un utilisateur, dans lequel la portion de surface de la portion d'au moins une dite partie surélevée, qui est jointe au corps de l'oreiller, est inférieure à la portion de surface de la surface supérieure de ladite au moins une partie surélevée, le corps comprenant, en plus des parties surélevées, une rainure continue disposée sur sa périphérie, la rainure étant disposée entre les surfaces supérieure et inférieure dudit corps.

2. Oreiller selon la revendication 1, dans lequel lesdites surfaces supérieure et inférieure dudit corps comportent chacune une pluralité desdites parties surélevées.

3. Oreiller selon l'une quelconque des revendications précédentes, dans lequel lesdites parties surélevées ont des coupes transversales sensiblement trapézoïdales inversées.

4. Oreiller selon l'une quelconque des revendications précédentes, dans lequel lesdites parties surélevées sont plus étroites à hauteur de cette partie d'au moins une dite partie surélevée qui est jointe au corps de l'oreiller qu'à hauteur de leur surface supérieure.

5. Oreiller selon l'une quelconque des revendications précédentes, dans lequel ledit oreiller comprend en outre une couverture faite dans du tissu.

6. Oreiller selon l'une quelconque des revendications précédentes, dans lequel ledit corps comprend une partie centrale et une première et une deuxième parties disposées de part et d'autre de ladite partie centrale,
**caractérisé en ce que**
au moins une portion de la partie centrale comprend une matière qui est moins déformable que la matière dans laquelle sont fabriquées la première et la deuxième parties.

7. Oreiller selon la revendication 6, dans lequel ladite partie centrale a une forme allongée et s'étend pratiquement sur la largeur de l'oreiller.

8. Oreiller selon, soit la revendication 6, soit la revendication7, dans lequel ladite partie centrale s'étend sur la hauteur de l'oreiller.

9. Oreiller selon l'une quelconque des revendications 6 à 8, dans lequel ladite partie centrale est disposée sur la surface supérieure du corps.

10. Oreiller selon l'une quelconque des revendications 6 à 9, dans lequel ladite partie centrale est disposée à l'intérieur du corps de l'oreiller.
